# EUROPEAN PATENT APPLICATION

(11) **EP 4 256 965 A1**
(43) Date of publication of application: **11.10.2023**
(21) Application number: 21900638.4
(22) Date of filing: 01.12.2021
(51) Int. Cl.: A23D 7/005, A23D 9/007

(54) **OIL-IN-WATER EMULSION COMPOSITION**

(30) Priority: 02.12.2020 JP 2020200450
(71) Applicant: Kao Corporation, Chuo-ku Tokyo 103-8210 (JP)
(72) Inventor: OUCHI, Mizue, Tokyo 131-8501 (JP); SAITO, Katsuyoshi, Tokyo 131-8501 (JP)
(74) Representative: Hoffmann Eitle
(86) International application number: PCT/JP2021/044104
(87) International publication number: WO 2022/118887

(57) **Abstract**

Provided is an oil-in-water emulsified composition including a diacylglycerol-containing fat or oil rich in α-linolenic acid and a vitamin C fatty acid ester and having less fish odor and fishy odor while having high oxidative stability. The oil-in-water emulsified composition includes the following components (A), (B) , and (C) : (A) a fat or oil having a content of diacylglycerols of 15 mass% or more and a content of α-linolenic acid in constituent fatty acids of the fat or oil of 15 mass% or more; (B) 0.020 mass% to 1.0 mass% of a vitamin C fatty acid ester with respect to the component (A); and (C) 0.15 mass% to 6.0 mass% of a hydroxy acid or a salt thereof.

## Description

### Field of the Invention

The present invention relates to an oil-in-water emulsified composition and a powdered fat or oil.

### Background of the Invention

It is known that α-linolenic acid (C18:3, ALA), which is rich in linseed and the like, is, when taken up into the body, converted into eicosapentaenoic acid (C20:5) or docosahexaenoic acid (C22:6), both of which have high physiological activity. In recent years, it has been desired that a fat or oil highly containing α-linolenic acid be utilized. However, the fat or oil rich in α-linolenic acid has extremely low oxidative stability and easily generates unpleasant odor. In view of the foregoing, in general, an antioxidant, such as tocopherol, a rosemary extract, an ascorbic acid fatty acid ester, catechin, or lecithin, is incorporated into the fat or oil to prevent deterioration of taste and flavor in the fat or oil.

Meanwhile, it has been reported that a fat or oil containing diacylglycerols at a high concentration has physiological actions, such as suppression of a postprandial increase in blood triglycerides (neutral fats) and a small accumulation amount in the body.

In view of the foregoing, in order to effectively exert physiologically active functions of α-linolenic acid and diacylglycerols, there have conventionally been proposed various functional fats or oils rich in α-linolenic acid and diacylglycerols (for example, Patent Document 1).

As the form of an edible fat or oil, there is given an oil-in-water (O/W) emulsion liquid or a powdered fat or oil obtained by drying and powdering the oil-in-water emulsion liquid. The powdered fat or oil is characterized by, for example, being quickly dissolved or dispersed in water, and can provide various effects, such as impartment in taste and flavor and improvement in texture, when used in a food.

For example, in Patent Document 2, there is a proposal of a powdered fat or oil, including: a glyceride mixture containing 15 wt% to 94.9 wt% of diglycerides, in which 50 wt% or more of all constituent fatty acids are unsaturated fatty acids; a powdered base; and water, and being excellent in, for example, taste and flavor and redispersibility in water. In Patent Document 3, there is a proposal of a powdered fat or oil, including: a highly unsaturated fatty acid-containing fat or oil; an edible protein; an edible carbohydrate; and an edible antioxidant, being excellent in, for example, water dispersibility, and less in oxidation deterioration odor after aging.
(Patent Document 1) JP-A-2002-138297
(Patent Document 2) JP-A-2003-306693
(Patent Document 3) JP-A-2019-41721

### Summary of the Invention

The present invention provides an oil-in-water emulsified composition, comprising the following components (A), (B), and (C) :
(A) a fat or oil having a content of diacylglycerols of 15 mass% or more and a content of α-linolenic acid in constituent fatty acids of the fat or oil of 15 mass% or more;
(B) 0.020 mass% to 1.0 mass% of a vitamin C fatty acid ester with respect to the component (A); and
(C) 0.15 mass% to 6.0 mass% of a hydroxy acid or a salt thereof.

Further, the present invention provides a powdered fat or oil, which is obtained by drying the oil-in-water emulsified composition.

### Detailed Description of the Invention

According to the present inventors, a vitamin C fatty acid ester is a preferred additive that can suppress the degradation of taste and flavor of a diacylglycerol-containing fat or oil rich in α-linolenic acid due to oxidation, in particular, light oxidation.

However, when an attempt was made to blend the diacylglycerol-containing fat or oil rich in α-linolenic acid and the vitamin C fatty acid ester to prepare an oil-in-water emulsified composition, while oxidative stability can be imparted to the oil-in-water emulsified composition, it found that fish odor and fishy odor, which were different from unpleasant odor generated through oxidation of the fat or oil, were generated immediately after preparing the oil-in-water emulsified composition.

Therefore, the present invention relates to the provision of an oil-in-water emulsified composition comprising a diacylglycerol-containing fat or oil rich in α-linolenic acid and a vitamin C fatty acid ester and having less fish odor and fishy odor while having high oxidative stability.

The present inventors found that, even in the case where the diacylglycerol-containing fat or oil rich in α-linolenic acid and the vitamin C fatty acid ester were used, when emulsification was performed after incorporation of a hydroxy acid or a salt thereof within a certain range, the oil-in-water emulsified composition and the powdered fat or oil having less fish odor and fishy odor while having oxidative stability were obtained.

According to the present invention, there can be provided the oil-in-water emulsified composition and the powdered fat or oil having high oxidative stability, having less fish odor and fishy odor, and having high functionalities of α-linolenic acid and diacylglycerols.

The oil-in-water emulsified composition of the present invention comprises (A) a fat or oil having a content of diacylglycerols of 15 mass% or more and a content of α-linolenic acid in constituent fatty acids of the fat or oil of 15 mass% or more. Herein, the fat or oil contains any one or more kinds of monoacylglycerols, diacylglycerols, and triacylglycerols. The kind of the fat or oil is not particularly limited, and may be any fat or oil that can be used as an edible fat or oil.

"(A) The fat or oil having a content of diacylglycerols of 15 mass% or more and a content of α-linolenic acid in constituent fatty acids of the fat or oil of 15 mass% or more" is hereinafter also referred to as "component (A) fat or oil."

In the present invention, the content of α-linolenic acid in the constituent fatty acids of the fat or oil of the component (A) fat or oil is 15 mass% (hereinafter simply referred to as "%") or more. The content of α-linolenic acid in the constituent fatty acids of the fat or oil is 15% or more, preferably 20% or more, more preferably 25% or more, more preferably 30% or more, more preferably 40% or more, more preferably 50% or more, even more preferably 52% or more, from the standpoint of physiological effects, and is preferably 70% or less, more preferably 65% or less, even more preferably 60% or less, from the standpoint of oxidative stability.

The content of α-linolenic acid in the constituent fatty acids of the fat or oil of the component (A) fat or oil is 15% or more, preferably from 15% to 70%, more preferably from 20% to 70%, more preferably from 25% to 70%, more preferably from 30% to 65%, more preferably from 40% to 65%, more preferably from 50% to 65%, even more preferably from 52% to 60%.

The amount of a fatty acid as used herein refers to an amount obtained by converting the fatty acid to a free fatty acid.

In the present invention, the constituent fatty acids of the component (A) fat or oil other than α-linolenic acid are not particularly limited, and may be any of saturated fatty acids and unsaturated fatty acids.

From the standpoints of the taste and flavor and industrial productivity of the fat or oil, the content of the unsaturated fatty acids in the constituent fatty acids of the component (A) fat or oil is preferably from 60% to 100%, more preferably from 70% to 100%, even more preferably from 80% to 99.5%. The carbon numbers of the unsaturated fatty acids are preferably from 14 to 24, more preferably from 16 to 22, from the standpoint of physiological effects.

In particular, the content of linoleic acid (C18:2) in the constituent fatty acids of the component (A) fat or oil is preferably 5% or more, more preferably 10% or more, and is preferably 40% or less, more preferably 30% or less, even more preferably 20% or less, from the standpoint of industrial productivity.

The content of linoleic acid in the constituent fatty acids of the component (A) fat or oil is preferably from 5% to 40%, more preferably from 5% to 30%, even more preferably from 10% to 20%.

In addition, the content of oleic acid (C18:1) in the constituent fatty acids of the component (A) fat or oil is preferably 10% or more, and is preferably 65% or less, more preferably 50% or less, even more preferably 30% or less, from the standpoint of industrial productivity.

The content of oleic acid in the constituent fatty acids of the component (A) fat or oil is preferably from 10% to 65%, more preferably from 10% to 50%, even more preferably from 10% to 30%.

In addition, the total content of the saturated fatty acids in the constituent fatty acids of the component (A) fat or oil is preferably 30% or less, more preferably 20% or less, more preferably 10% or less, even more preferably 7% or less, and is preferably 0.5% or more, from the standpoints of appearance, physiological effects, and the industrial productivity of the fat or oil.

The carbon numbers of the saturated fatty acids are preferably from 14 to 24, more preferably from 16 to 22.

In the present invention, the component (A) fat or oil contains diacylglycerols in a content of 15% or more. As the concentration of the diacylglycerols in the component (A) fat or oil becomes a higher concentration, incorporation of (B) a vitamin C fatty acid ester becomes easier, and thus oxidative stability can be enhanced. Accordingly, it is preferred that the diacylglycerols be incorporated into the fat or oil at a high content from the standpoint of the effective exertion of effects.

The content of the diacylglycerols in the component (A) fat or oil is 15% or more, preferably 20% or more, more preferably 25% or more, more preferably 30% or more, more preferably 50% or more, more preferably 60% or more, more preferably 70% or more, even more preferably 80% or more, from the standpoints of the solubility of the vitamin C fatty acid ester, appearance, and physiological effects, and is preferably 99.5% or less, more preferably 98% or less, more preferably 95% or less, even more preferably 90% or less, from the standpoint of industrial productivity.

The content of the diacylglycerols in the component (A) fat or oil is 15% or more, preferably from 15% to 99.5%, more preferably from 20% to 98%, more preferably from 25% to 98%, more preferably from 30% to 95%, more preferably from 50% to 95%, more preferably from 60% to 90%, more preferably from 70% to 90%, even more preferably from 80% to 90%.

The component (A) fat or oil preferably contains triacylglycerols, and the content of the triacylglycerols in the component (A) fat or oil is preferably 1% or more, more preferably 2% or more, more preferably 5% or more, even more preferably 10% or more, and is preferably 85% or less, more preferably 80% or less, more preferably 75% or less, more preferably 72% or less, more preferably 50% or less, even more preferably 25% or less, from the standpoint of the industrial productivity of the fat or oil.

The content of the triacylglycerols in the component (A) fat or oil is preferably from 1% to 85%, more preferably from 2% to 80%, more preferably from 2% to 75%, more preferably from 5% to 50%, even more preferably from 10% to 25%.

The content of monoacylglycerols in the component (A) fat or oil is preferably 5% or less, more preferably 3% or less, more preferably 2% or less, even more preferably 1.5% or less, and is preferably more than 0%, from the standpoints of taste and flavor, the industrial productivity of the fat or oil, and oxidative stability. The content of the monoacylglycerols in the fat or oil may be 0%.

The content of a free fatty acid or a salt thereof in the component (A) fat or oil is preferably 3% or less, more preferably 2% or less, even more preferably 1% or less, and is preferably more than 0%, from the standpoints of taste and flavor and oxidative stability. The content of the free fatty acid or the salt thereof in the fat or oil may be 0%.

The fatty acid compositions of the triacylglycerols, the diacylglycerols, and the monoacylglycerols may be identical to or different from each other.

The component (A) fat or oil may be obtained, for example, by: an esterification reaction between fatty acids derived from a fat or oil, and glycerin; or a transesterification reaction (glycerolysis) between a fat or oil, and glycerin.

The esterification reaction and the glycerolysis reaction are broadly classified into: a chemical method involving using a chemical catalyst, such as an alkali metal or an alloy thereof, an oxide or hydroxide of an alkali metal or an alkaline earth metal, or an alkoxide of an alkali metal or an alkaline earth metal; and an enzymatic method involving using an enzyme such as a lipase.

Of those, an esterification reaction between fractionated fatty acids derived from a fat or oil, which are described later, and glycerin is preferred from the standpoint of controlling fatty acid composition.

In the present invention, the fat or oil (edible fat or oil) may be any of a plant-derived fat or oil and an animal-derived fat or oil. Examples thereof may include fats or oils including: plant-derived fats or oils, such as soybean oil, rapeseed oil, safflower oil, rice oil, corn oil, sunflower oil, cotton seed oil, olive oil, sesame oil, peanut oil, Job's tears seed oil, wheat germ oil, Japanese basil oil, linseed oil (flaxseed oil), perilla oil, chia seed oil, sacha inchi oil, walnut oil, kiwi seed oil, salvia seed oil, grape seed oil, macadamia nut oil, hazelnut oil, pumpkin seed oil, camellia oil, tea seed oil, borage oil, palm oil, palm olein, palm stearin, coconut oil, palm kernel oil, cacao butter, sal butter, shea butter, and algae oil; animal-derived fats or oils, such as fish oil, lard, beef tallow, and butter fat; and transesterified oils, hydrogenated oils, and fractionated oils thereof.

Of those, from the standpoint of usability, a plant-derived fat or oil is preferably used, a liquid fat or oil excellent in low-temperature resistance is more preferably used, and at least one kind of fat or oil selected from the group consisting of Japanese basil oil, linseed oil (flaxseed oil), perilla oil, chia seed oil, and sacha inchi oil rich in α-linolenic acid is even more preferably used. The liquid fat or oil means a fat or oil that is in a liquid state at 20°C, when determined in accordance with a cold test described in Standard Methods for Analysis of Fats, Oils and Related Materials 2.3.8-27.

The fatty acids derived from a fat or oil may be obtained by hydrolyzing a fat or oil. As a method of hydrolyzing the fat or oil, there are given a high-temperature and high-pressure degradation method and an enzymatic degradation method. The high-temperature and high-pressure degradation method is a method involving adding water to a fat or oil, and subjecting the mixture to a reaction under high-temperature and high-pressure conditions, to thereby provide fatty acids and glycerin. In addition, the enzymatic degradation method is a method involving adding water to a fat or oil, and subjecting the mixture to a reaction under a low-temperature condition using a fat or oil hydrolytic enzyme as a catalyst, to thereby provide fatty acids and glycerin.

The hydrolysis reaction may be performed in accordance with a conventional method.

After the hydrolysis of the fat or oil, it is preferred that a hydrolysate be fractionated and a solid be removed. As a fractionation method, there are given a solvent fractionation method, a spontaneous fractionation method (dry fractionation method), and a wetting agent fractionation method.

As means for removing a precipitated solid, there are given, for example, settled separation, filtration, centrifugation, and a method involving mixing fatty acids with an aqueous solution of a wetting agent, followed by separation.

The esterification reaction between the fatty acids derived from a fat or oil, and glycerin is preferably performed by an enzymatic method under mild conditions, from the standpoint of, for example, excellent taste and flavor.

The usage amount of the enzyme may be appropriately decided in consideration of the activity of the enzyme. From the standpoint of increasing a reaction rate, when an immobilized enzyme is used, the usage amount is preferably from 1% to 30%, more preferably from 2% to 20%, with respect to the total mass of raw materials for the esterification reaction.

The reaction temperature of the esterification reaction is preferably from 0°C to 100°C, more preferably from 20°C to 80°C, even more preferably from 30°C to 60°C, from the standpoint of increasing a reaction rate and the standpoint of suppressing the inactivation of the enzyme. In addition, a reaction time is preferably 15 hours or less, more preferably from 1 hour to 12 hours, even more preferably from 2 hours to 10 hours, from the standpoint of industrial productivity.

As means for bringing the fatty acids and glycerin into contact with each other, there are given, for example, immersion, stirring, and a method involving passing a liquid through a column packed with an immobilized lipase through use of a pump or the like.

After the esterification reaction, a refinement process to be generally used for a fat or oil may be performed. Specific examples thereof may include processes, such as distillation treatment, acid treatment, water washing, decoloration, and deodorization.

The content of the component (A) fat or oil in the oil-in-water emulsified composition is preferably 5% or more, more preferably 8% or more, more preferably 10% or more, more preferably 15% or more, more preferably 20% or more, even more preferably 30% or more, and is preferably 80% or less, more preferably 75% or less, more preferably 70% or less, more preferably 60% or less, more preferably 50% or less, even more preferably 45% or less, from the standpoints of emulsion stability, oxidative stability, taste and flavor, and industrial productivity.

The content of the component (A) fat or oil in the oil-in-water emulsified composition is preferably from 5% to 80%, more preferably from 8% to 75%, more preferably from 10% to 70%, more preferably from 15% to 60%, more preferably from 20% to 50%, even more preferably from 30% to 45%.

The oil-in-water emulsified composition of the present invention comprises (B) a vitamin C fatty acid ester. The "vitamin C fatty acid ester" is hereinafter also referred to as "component (B) ".

The vitamin C fatty acid ester used in the present invention is an ester of L-ascorbic acid and a higher fatty acid, and a commercially available product, preferably a commercially available product for a food and drink may be used. The higher fatty acid is preferably a linear saturated fatty acid having 12 to 22 carbon atoms. The vitamin C fatty acid esters may be used alone or in combination thereof.

The vitamin C fatty acid ester is preferably vitamin C palmitate, vitamin C stearate, or combinations thereof, more preferably vitamin C palmitate, from the standpoint of having high oxidative stability and the standpoint of having less fish odor and fishy odor and satisfactory taste and flavor.

In the present invention, the content of the component (B) in the oil-in-water emulsified composition with respect to the component (A) is from 0.020% to 1.0%. The content of the component (B) in the oil-in-water emulsified composition with respect to the component (A) is 0.020% or more, preferably 0.025% or more, more preferably 0.03% or more, more preferably 0.05% or more, even more preferably 0.1% or more, from the standpoint of oxidative stability, and is 1.0% or less, preferably 0.5% or less, more preferably 0.3% or less, even more preferably 0.2% or less, from the standpoints of the solubility of the vitamin C fatty acid ester and appearance.

The content of the component (B) in the oil-in-water emulsified composition with respect to the component (A) is from 0.020% to 1.0%, preferably from 0.025% to 1.0%, more preferably from 0.03% to 0.5%, more preferably from 0.05% to 0.5%, more preferably from 0.1% to 0.5%, more preferably from 0.1% to 0.3%, even more preferably from 0.1% to 0.2%.

The oil-in-water emulsified composition of the present invention comprises (C) a hydroxy acid or a salt thereof. The "hydroxy acid or the salt thereof" is hereinafter also referred to as "component (C)".

The hydroxy acid to be used in the present invention is a collective term for compounds each having a carboxy group and an alcoholic hydroxy group in a molecule thereof, and examples thereof include lactic acid, tartaric acid, malic acid, and citric acid.

The salt of the hydroxy acid is preferably an alkali metal salt, more preferably a sodium salt and a potassium salt, even more preferably a sodium salt. Those salts may be used alone or in combination thereof. The hydroxy acid or the salt thereof may be any of an anhydride and a hydrate.

Of those, one or more selected from the group consisting of citric acid, tartaric acid, malic acid, and salts thereof are each preferred as the hydroxy acid or the salt thereof, citric acid or sodium citrate is more preferred, and sodium citrate is even more preferred, from the standpoints of oxidative stability and taste and flavor.

In the present invention, the content of the component (C) in the oil-in-water emulsified composition is from 0.15% to 6.0%. The content of the component (C) in the oil-in-water emulsified composition is 0.15% or more, preferably 0.20% or more, more preferably 0.30% or more, more preferably 0.50% or more, even more preferably 0.60% or more, from the standpoints of oxidative stability and taste and flavor, and is 6.0% or less, preferably 4.0% or less, more preferably 3.0% or less, more preferably 2.0% or less, even more preferably 1.0% or less, from the standpoints of oxidative stability and taste and flavor.

The content of the component (C) in the oil-in-water emulsified composition is from 0.15% to 6.0%, preferably from 0.20% to 4.0%, more preferably from 0.30% to 3.0%, more preferably from 0.50% to 2.0%, even more preferably from 0.60% to 1.0%.

Herein, when the component (C) is a salt or a hydrate, the content of the component (C) refers to a value obtained by converting the content into the content of the hydroxy acid serving as a free acid.

The oil-in-water emulsified composition of the present invention preferably further comprises (D) an emulsifying agent, from the standpoints of emulsion stability, appearance, and industrial productivity. The "emulsifying agent" is hereinafter also referred to as "component (D)".

Examples of the emulsifying agent to be used in the present invention include: processed starch; synthetic emulsifying agents, such as an organic acid monoglyceride, a polyglycerin fatty acid ester, a propylene glycol fatty acid ester, a polyglycerin condensed ricinoleic acid ester, a sorbitan fatty acid ester, a sorbitol fatty acid ester, and a sucrose fatty acid ester; and natural emulsifying agents, such as saponin, lecithin or an enzymatic degradation product thereof, and proteins, such as milk proteins (e.g., butter milk powder and casein sodium), a soy protein, and a wheat protein, or isolates or degradation products of those proteins. Those emulsifying agents may be used alone or in combination thereof. Of those, one or more selected from the group consisting of processed starch, a sucrose fatty acid ester, a polyglycerin fatty acid ester, lecithin or an enzymatic degradation product thereof, and milk proteins are preferred, one or more selected from the group consisting of processed starch, a polyglycerin fatty acid ester, and milk proteins are more preferred, and one or more selected from the group consisting of milk proteins are even more preferred, from the standpoints of emulsion stability, taste and flavor, and oxidative stability.

Examples of the processed starch include starches subjected to processes including: etherification, such as hydroxypropyl starch and carboxymethyl starch; esterification, such as acetylated starch, octenylsuccinic acid starch, and phosphoric acid starch; cross-linking, such as phosphoric acid cross-linked starch and adipic acid cross-linked starch; and oxidation such as an oxidized starch treated with an oxidant such as sodium hypochlorite; or a combination of those processes.

The content of the component (D) in the oil-in-water emulsified composition is preferably 0.05% or more, more preferably 0.2% or more, more preferably 0.5% or more, even more preferably 1% or more, and is preferably 30% or less, more preferably 10% or less, even more preferably 5% or less, from the standpoints of emulsion stability, taste and flavor, and oxidative stability.

The content of the component (D) in the oil-in-water emulsified composition is preferably from 0.05% to 30%, more preferably from 0.2% to 10%, more preferably from 0.5% to 10%, even more preferably from 1% to 5%.

In the present invention, a mass ratio between the content of the component (B) with respect to the component (A) and the content of α-linolenic acid in the constituent fatty acids of the component (A) fat or oil, [(ALA)/(component (B))], is preferably 30 or more, more preferably 40 or more, more preferably 50 or more, more preferably 100 or more, more preferably 150 or more, even more preferably 200 or more, from the standpoints of taste and flavor, the solubility of the vitamin C fatty acid ester, physiological functions, and oxidative stability, and is preferably 3, 000 or less, more preferably 2, 500 or less, more preferably 2,000 or less, more preferably 1, 500 or less, more preferably 1,000 or less, even more preferably 800 or less, from the standpoints of taste and flavor and oxidative stability.

In the present invention, a mass ratio between the content of the component (B) with respect to the component (A) and the content of α-linolenic acid in the constituent fatty acids of the component (A) fat or oil, [(ALA)/(component (B))], is preferably from 30 to 3, 000, more preferably from 40 to 2,500, more preferably from 50 to 2,000, more preferably from 100 to 1,500, more preferably from 150 to 1,000, even more preferably from 200 to 800, from the standpoints of taste and flavor, the solubility of the vitamin C fatty acid ester, physiological functions, and oxidative stability.

In the present invention, a mass ratio between the content of the component (B) with respect to the component (A) and the content of the component (C) in the oil-in-water emulsified composition, [(component (C)) / (component (B))], is preferably 0.20 or more, more preferably 0.30 or more, more preferably 0.50 or more, more preferably 2.0 or more, more preferably 3.0 or more, even more preferably 5.0 or more, from the standpoints of taste and flavor and oxidative stability, and is preferably 200 or less, more preferably 100 or less, more preferably 50 or less, more preferably 40 or less, more preferably 30 or less, even more preferably 20 or less, from the standpoints of taste and flavor and oxidative stability.

In the present invention, a mass ratio between the content of the component (B) with respect to the component (A) and the content of the component (C) in the oil-in-water emulsified composition, [(component (C))/(component (B))], is preferably from 0.20 to 200, more preferably from 0.30 to 100, more preferably from 0.50 to 50, more preferably from 0.50 to 40, more preferably from 0.50 to 30, more preferably from 2.0 to 30, more preferably from 3.0 to 30, even more preferably from 5.0 to 20, from the standpoints of taste and flavor and oxidative stability.

An antioxidant except the component (B) or any other oil-soluble component may be incorporated into an oil phase of the oil-in-water emulsified composition, from the standpoint of the oxidative stability of the component (A) fat or oil. The content of the oil-soluble component may be appropriately set within a range in which the purpose of the present invention is not impaired.

The aqueous phase of the oil-in-water emulsified composition is not particularly limited, and a water-soluble component, such as: water; an excipient, such as a monosaccharide, a disaccharide, a starch degradation product, or a sugar alcohol; a hydrophilic antioxidant; a thickener; an antiseptic; a colorant; a sweetener; a seasoning; or a flavor may be incorporated into the aqueous phase. In the present invention, those components may be blended as appropriate in accordance with, for example, the viscosity or physical properties of the target composition.

The pH of the aqueous phase is preferably from 1 to 12, more preferably from 2 to 11, even more preferably from 5 to 10, from the standpoints of oxidative stability, emulsion stability, and taste and flavor.

A blending ratio (mass ratio) between the oil phase and the aqueous phase in the oil-in-water emulsified composition is preferably from 5/95 to 80/20, more preferably from 8/92 to 75/25, more preferably from 10/90 to 70/30, more preferably from 15/85 to 60/40, more preferably from 20/80 to 50/50, even more preferably from 30/70 to 45/55.

The oil-in-water emulsified composition of the present invention may be produced by mixing the component (A), the component (B), and the component (C), and the component (D) as required in accordance with a conventional method. It is preferred that an oil phase be first prepared by mixing the component (A) and the component (B) . Meanwhile, water and the component (C), and the component (D) as required are mixed to prepare an aqueous phase. Next, the oil phase is added to the aqueous phase, and preliminary emulsification is performed as required to homogenize the composition. Thus, an oil-in-water emulsified composition can be obtained.

As a homogenizer, there are given, for example, a high-pressure homogenizer, an ultrasonic emulsifier, a colloid mill, Agihomomixer, and a milder.

The oil-in-water emulsified composition thus produced may be used as it is, and may also be dried and used as a powdered fat or oil. The oil-in-water emulsified composition of the present invention has high oxidative stability and less fish odor and fishy odor while containing a large amount of α-linolenic acid, and hence a powdered fat or oil containing a large amount of α-linolenic acid and having satisfactory taste and flavor can be provided.

A general method may be used as a drying method. Examples thereof include spray drying, freeze drying, vacuum drying, belt drying, shelf drying, and drum drying. For example, classification, granulation, or pulverization may be performed as required so that particles each having a desired particle diameter are obtained. Of those, spray drying is preferred from the standpoints of productivity, thermal history, and a particle shape.

The oil-in-water emulsified composition and powdered fat or oil of the present invention may each be used in the same manner as a general edible fat or oil, and may be applied to various foods and drinks and feeds each using a fat or oil. Examples of the food and drink include foods for specified health use and foods with function claims claiming to have the physiological effects of α-linolenic acid and diacylglycerols as well as general foods and drinks.

The form of the food and drink may be a solid, a semisolid, or a liquid, and examples thereof include a drink, a water-in-oil fat or oil-containing food, an oil-in-water fat or oil-containing food, a bakery food, confectionery, a frozen food, a retort food, and a composition for nutritional supplementation, such as a tablet, a capsule, or a troche.

Examples of the feed include: a livestock feed to be used for cattle, swine, or the like; a small-animal feed to be used for rabbits, mice, or the like; a fish and shellfish feed to be used for eels, prawns, or the like; and a pet food to be used for dogs, cats, or the like.

The oil-in-water emulsified composition of the present invention is preferably an oil-in-water emulsified composition comprising the following components (A), (B), and (C), from the standpoint of having high oxidative stability, the standpoint of having less fish odor and fishy odor and satisfactory taste and flavor, and the standpoint of physiological effects:
(A) a fat or oil having a content of diacylglycerols of from 20 mass% to 98 mass% and a content of α-linolenic acid in constituent fatty acids of the fat or oil of from 20 mass% to 70 mass%;
(B) 0.025 mass% to 1.0 mass% of a vitamin C fatty acid ester with respect to the component (A); and
(C) 0.20 mass% to 6.0 mass% of a hydroxy acid or a salt thereof.

The oil-in-water emulsified composition of the present invention is preferably an oil-in-water emulsified composition comprising the following components (A), (B), (C), and (D), from the standpoint of having high oxidative stability, the standpoint of having less fish odor and fishy odor and satisfactory taste and flavor, and the standpoint of physiological effects:
(A) a fat or oil having a content of diacylglycerols of from 20 mass% to 98 mass% and a content of α-linolenic acid in constituent fatty acids of the fat or oil of from 20 mass% to 70 mass%;
(B) 0.025 mass% to 1.0 mass% of a vitamin C fatty acid ester with respect to the component (A);
(C) 0.20 mass% to 6.0 mass% of a hydroxy acid or a salt thereof; and
(D) 0.05 mass% to 30 mass% of an emulsifying agent.

The oil-in-water emulsified composition of the present invention is preferably an oil-in-water emulsified composition comprising the following components (A), (B), (C), and (D), from the standpoint of having high oxidative stability, the standpoint of having less fish odor and fishy odor and satisfactory taste and flavor, and the standpoint of physiological effects:
(A) a fat or oil having a content of diacylglycerols of from 20 mass% to 95 mass% and a content of α-linolenic acid in constituent fatty acids of the fat or oil of from 20 mass% to 70 mass%;
(B) 0.025 mass% to 0.5 mass% of a vitamin C fatty acid ester with respect to the component (A);
(C) 0.20 mass% to 6.0 mass% of a hydroxy acid or a salt thereof; and
(D) 0.2 mass% to 30 mass% of an emulsifying agent.

The oil-in-water emulsified composition of the present invention is preferably an oil-in-water emulsified composition comprising the following components (A), (B), (C), and (D), from the standpoint of having high oxidative stability, the standpoint of having less fish odor and fishy odor and satisfactory taste and flavor, and the standpoint of physiological effects:
(A) a fat or oil having a content of diacylglycerols of from 30 mass% to 90 mass% and a content of α-linolenic acid in constituent fatty acids of the fat or oil of from 25 mass% to 70 mass%;
(B) 0.05 mass% to 0.5 mass% of a vitamin C fatty acid ester with respect to the component (A);
(C) 0.20 mass% to 3.0 mass% of a hydroxy acid or a salt thereof; and
(D) 0.2 mass% to 10 mass% of an emulsifying agent.

In relation to the above-mentioned embodiments, the present invention further discloses the following oil-in-water emulsified compositions and powdered fats or oils.
<1> An oil-in-water emulsified composition, comprising the following components (A), (B), and (C) :
   (A) a fat or oil having a content of diacylglycerols of 15 mass% or more and a content of α-linolenic acid in constituent fatty acids of the fat or oil of 15 mass% or more;
   (B) 0.020 mass% to 1.0 mass% of a vitamin C fatty acid ester with respect to the component (A); and
   (C) 0.15 mass% to 6.0 mass% of a hydroxy acid or a salt thereof.
<2> The oil-in-water emulsified composition according to Item <1>, wherein the content of α-linolenic acid in the constituent fatty acids of the component (A) fat or oil is 15 mass% or more, preferably 20 mass% or more, more preferably 25 mass% or more, more preferably 30 mass% or more, more preferably 40 mass% or more, more preferably 50 mass% or more, even more preferably 52 mass% or more, is preferably 70 mass% or less, more preferably 65 mass% or less, even more preferably 60 mass% or less, and is preferably from 15 mass% to 70 mass%, more preferably from 20 mass% to 70 mass%, more preferably from 25 mass% to 70 mass%, more preferably from 30 mass% to 65 mass%, more preferably from 40 mass% to 65 mass%, more preferably from 50 mass% to 65 mass%, even more preferably from 52 mass% to 60 mass%.
<3> The oil-in-water emulsified composition according to Item <1> or <2>, wherein the content of linoleic acid in the constituent fatty acids of the fat or oil of the component (A) fat or oil is preferably 5 mass% or more, more preferably 10 mass% or more, is preferably 40 mass% or less, more preferably 30 mass% or less, even more preferably 20 mass% or less, and is preferably from 5 mass% to 40 mass%, more preferably from 5 mass% to 30 mass%, even more preferably from 10 mass% to 20 mass%.
<4> The oil-in-water emulsified composition according to any one of Items <1> to <3>, wherein the content of oleic acid in the constituent fatty acids of the fat or oil of the component (A) fat or oil is preferably 10 mass% or more, is preferably 65 mass% or less, more preferably 50 mass% or less, even more preferably 30 mass% or less, and is preferably from 10 mass% to 65 mass%, more preferably from 10 mass% to 50 mass%, even more preferably from 10 mass% to 30 mass%.
<5> The oil-in-water emulsified composition according to any one of Items <1> to <4>, wherein the content of the diacylglycerols in the component (A) fat or oil is 15 mass% or more, preferably 20 mass% or more, more preferably 25 mass% or more, more preferably 30 mass% or more, more preferably 50 mass% or more, more preferably 60 mass% or more, more preferably 70 mass% or more, even more preferably 80 mass% or more, is preferably 99.5 mass% or less, more preferably 98 mass% or less, more preferably 95 mass% or less, even more preferably 90 mass% or less, and is preferably from 15 mass% to 99.5 mass%, more preferably from 20 mass% to 98 mass%, more preferably from 25 mass% to 98 mass%, more preferably from 30 mass% to 95 mass%, more preferably from 50 mass% to 95 mass%, more preferably from 60 mass% to 90 mass%, more preferably from 70 mass% to 90 mass%, even more preferably from 80 mass% to 90 mass%.
<6> The oil-in-water emulsified composition according to any one of Items <1> to <5>, wherein the content of triacylglycerols in the component (A) fat or oil is preferably 1 mass% or more, more preferably 2 mass% or more, more preferably 5 mass% or more, even more preferably 10 mass% or more, is preferably 85 mass% or less, more preferably 80 mass% or less, more preferably 75 mass% or less, more preferably 50 mass% or less, even more preferably 25 mass% or less, and is preferably from 1 mass% to 85 mass%, more preferably from 2 mass% to 80 mass%, more preferably from 2 mass% to 75 mass%, more preferably from 5 mass% to 50 mass%, even more preferably from 10 mass% to 25 mass%.
<7> The oil-in-water emulsified composition according to any one of Items <1> to <6>, wherein the content of monoacylglycerols in the component (A) fat or oil is preferably 5 mass% or less, more preferably 3 mass% or less, more preferably 2 mass% or less, even more preferably 1.5 mass% or less, and is preferably more than 0 mass%.
<8> The oil-in-water emulsified composition according to any one of Items <1> to <7>, wherein the content of a free fatty acid or a salt thereof in the component (A) fat or oil is preferably 3 mass% or less, more preferably 2 mass% or less, even more preferably 1 mass% or less, and is preferably more than 0 mass%.
<9> The oil-in-water emulsified composition according to any one of Items <1> to <8>, wherein the component (A) fat or oil preferably contains an oil obtained by an esterification reaction between fractionated fatty acids derived from a fat or oil, and glycerin.
<10> The oil-in-water emulsified composition according to Item <9>, wherein the fractionated fatty acids are preferably derived from at least one kind of fat or oil selected from the group consisting of Japanese basil oil, linseed oil (flaxseed oil), perilla oil, chia seed oil, and sacha inchi oil.
<11> The oil-in-water emulsified composition according to any one of Items <1> to <10>, wherein the content of the component (A) fat or oil in the oil-in-water emulsified composition is preferably 5 mass% or more, more preferably 8 mass% or more, more preferably 10 mass% or more, more preferably 15 mass% or more, more preferably 20 mass% or more, even more preferably 30 mass% or more, is preferably 80 mass% or less, more preferably 75 mass% or less, more preferably 70 mass% or less, more preferably 60 mass% or less, more preferably 50 mass% or less, even more preferably 45 mass% or less, and is preferably from 5 mass% to 80 mass%, more preferably from 8 mass% to 75 mass%, more preferably from 10 mass% to 70 mass%, more preferably from 15 mass% to 60 mass%, more preferably from 20 mass% to 50 mass%, even more preferably from 30 mass% to 45 mass%.
<12> The oil-in-water emulsified composition according to any one of Items <1> to <11>, wherein the component (B) is preferably vitamin C palmitate, vitamin C stearate, or combinations thereof, more preferably vitamin C palmitate.
<13> The oil-in-water emulsified composition according to any one of Items <1> to <12>, wherein the content of the component (B) in the oil-in-water emulsified composition with respect to the component (A) is 0.020 mass% or more, preferably 0.025 mass% or more, more preferably 0.03 mass% or more, more preferably 0.05 mass% or more, even more preferably 0.1 mass% or more, is 1.0 mass% or less, preferably 0.5 mass% or less, more preferably 0.3 mass% or less, even more preferably 0.2 mass% or less, and is from 0.020 mass% to 1.0 mass%, preferably from 0.025 mass% to 1.0 mass%, more preferably from 0.03 mass% to 0.5 mass%, more preferably from 0.05 mass% to 0.5 mass%, more preferably from 0.1 mass% to 0.5 mass%, more preferably from 0.1 mass% to 0.3 mass%, even more preferably from 0.1 mass% to 0.2 mass%.
<14> The oil-in-water emulsified composition according to any one of Items <1> to <13>, wherein (C) the hydroxy acid or the salt thereof is preferably one or more selected from the group consisting of citric acid, tartaric acid, malic acid, and salts thereof, more preferably citric acid, sodium citrate, or combinations thereof, even more preferably sodium citrate.
<15> The oil-in-water emulsified composition according to any one of Items <1> to <14>, wherein the content of the component (C) in the oil-in-water emulsified composition is 0.15 mass% or more, preferably 0.20 mass% or more, more preferably 0.30 mass% or more, more preferably 0.50 mass% or more, even more preferably 0.60 mass% or more, is 6.0 mass% or less, preferably 4.0 mass% or less, more preferably 3.0 mass% or less, more preferably 2.0 mass% or less, even more preferably 1.0 mass% or less, and is from 0.15 mass% to 6.0 mass%, preferably from 0.20 mass% to 4.0 mass%, more preferably from 0.30 mass% to 3.0 mass%, more preferably from 0.50 mass% to 2.0 mass%, even more preferably from 0.60 mass% to 1.0 mass%.
<16> The oil-in-water emulsified composition according to any one of Items <1> to <15>, preferably further comprising (D) an emulsifying agent.
<17> The oil-in-water emulsified composition according to Item <16>, wherein (D) the emulsifying agent is preferably one or more selected from the group consisting of processed starch, a sucrose fatty acid ester, a polyglycerin fatty acid ester, lecithin or an enzymatic degradation product thereof, and milk proteins, more preferably one or more selected from the group consisting of processed starch, a polyglycerin fatty acid ester, and milk proteins, even more preferably one or more selected from the group consisting of milk proteins.
<18> The oil-in-water emulsified composition according to Item <16> or <17>, wherein the content of the component (D) in the oil-in-water emulsified composition is preferably 0.05 mass% or more, more preferably 0.2 mass% or more, more preferably 0.5 mass% or more, even more preferably 1 mass% or more, is preferably 30 mass% or less, more preferably 10 mass% or less, even more preferably 5 mass% or less, and is preferably from 0.05 mass% to 30 mass%, more preferably from 0.2 mass% to 10 mass%, more preferably from 0.5 mass% to 10 mass%, even more preferably from 1 mass% to 5 mass%.
<19> The oil-in-water emulsified composition according to any one of Items <1> to <18>, wherein a mass ratio between the content of the component (B) with respect to the component (A) and the content of α-linolenic acid in the constituent fatty acids of the component (A) fat or oil, [(ALA)/(component (B))], is preferably 30 or more, more preferably 40 or more, more preferably 50 or more, more preferably 100 or more, more preferably 150 or more, even more preferably 200 or more, is preferably 3,000 or less, more preferably 2,500 or less, more preferably 2,000 or less, more preferably 1,500 or less, more preferably 1,000 or less, even more preferably 800 or less, and is preferably from 30 to 3,000, more preferably from 40 to 2,500, more preferably from 50 to 2,000, more preferably from 100 to 1,500, more preferably from 150 to 1,000, even more preferably from 200 to 800.
<20> The oil-in-water emulsified composition according to any one of Items <1> to <19>, wherein a mass ratio between the content of the component (B) with respect to the component (A) and the content of the component (C) in the oil-in-water emulsified composition, [(component (C))/(component (B))], is preferably 0.20 or more, more preferably 0.30 or more, more preferably 0.50 or more, more preferably 2.0 or more, more preferably 3.0 or more, even more preferably 5.0 or more, is preferably 200 or less, more preferably 100 or less, more preferably 50 or less, more preferably 40 or less, more preferably 30 or less, even more preferably 20 or less, and is preferably from 0.20 to 200, more preferably from 0.30 to 100, more preferably from 0.50 to 50, more preferably from 0.50 to 40, more preferably from 0.50 to 30, more preferably from 2.0 to 30, more preferably from 2.0 to 30, more preferably from 3.0 to 30, even more preferably from 5.0 to 20.
<21> The oil-in-water emulsified composition according to any one of Items <1> to <20>, wherein a blending ratio (mass ratio) between the oil phase and the aqueous phase is preferably from 5/95 to 80/20, more preferably from 8/92 to 75/25, more preferably from 10/90 to 70/30, more preferably from 15/85 to 60/40, more preferably from 20/80 to 50/50, even more preferably from 30/70 to 45/55.
<22> A powdered fat or oil, which is obtained by drying the oil-in-water emulsified composition of any one of Items <1> to <21>.
<23> A food and drink, comprising the oil-in-water emulsified composition of any one of Items <1> to <21> or the powdered fat or oil of Item <22>.
<24> A feed, comprising the oil-in-water emulsified composition of any one of Items <1> to <21> or the powdered fat or oil of Item <22>.

### Examples

### [Analysis Method]

### (i) Composition of Glycerides in Fat or oil

About 10 mg of a fat or oil sample and 0.5 mL of a trimethylsilylating agent ("Silylating agent TH," manufactured by Kanto Chemical Co., Inc.) were placed in a glass sample bottle, and the bottle was sealed and heated at 70°C for 15 minutes. 1.0 mL of water and 1.5 mL of hexane were added thereto, and the bottle was shaken. The bottle was left at rest, and then the upper layer was analyzed by gas-liquid chromatography (GLC).

### <GLC Analysis Conditions>

### (Conditions)

Apparatus: Agilent 7890B (manufactured by Agilent Technologies) Column: DB-1ht 10 m × 0.25 mm × 0.2 µm (manufactured by Agilent J&W)
Carrier gas: 1.0 mL He/min
Injector: Split (1:50), T=340°C
Detector: FID, T=350°C
Oven temperature: The temperature was increased from 80°C at 10°C/min to 340°C, and kept for 15 minutes.

### (ii) Composition of Constituent Fatty Acids of Fat or oil

Fatty acid methyl esters were prepared in accordance with "Preparation method for fatty acid methyl ester (2.4.1.-1996)" described in "Standard Method for the Analysis of Fats, Oils and Related Materials" edited by Japan Oil Chemists' Society, and the resultant fat or oil samples were subjected to measurement in conformity with American Oil Chemists. Society Official Method Ce 1f-96 (GLC method).

### <GLC Analysis Conditions>

Apparatus: Agilent 7890B (manufactured by Agilent Technologies)
Column: CP-SIL88 50 m × 0.25 mm × 0.2 um (manufactured by Agilent J&W)
Carrier gas: 1.0 mL He/min
Injector: Split (1:50), T=300°C
Detector: FID, T=300°C
Oven temperature: kept at 150°C for 5 min→increased at 1°C/min→kept at 160°C for 5 min → increased at 2°C/min → kept at 200°C for 10 min → increased at 10°C/min → kept at 220°C for 5 min

### (iii) Measurement of Peroxide Value (POV)

5 mL of deionized water was added to about 2.5 g of an oil-in-water emulsified composition, followed by stirring. After that, 10 mL of THF was added thereto, and then the resultant was stirred and was subjected to centrifugation (3,000 r/min, 10 minutes, 20°C) . Next, 20 mL of hexane and 10 mL of ion-exchanged water were added thereto and the resultant was subjected to centrifugation (3,000 r/min, 5 minutes, 20°C). After that, the upper layer was taken out and the solvent was evaporated with a rotary evaporator. Thus, a fat or oil was prepared.

The POV of the extracted fat or oil was measured based on the JOCS Standard Methods for the Analysis of Fats, Oils and Related Materials 2.5.2.2-2013.

### [Raw Material]

The following fat or oil "a" to fat or oil "d" were prepared.

Fat or oil "a": Fatty acids obtained by hydrolyzing semi-refined linseed oil (manufactured by ADM) with an enzyme were winterized to reduce the content of saturated fatty acids. Then, the fractionated fatty acids and glycerin were subjected to an esterification reaction under reduced pressure through use of a commercially available immobilized 1,3-selective lipase as a catalyst. The immobilized enzyme was separated by filtration, and then the product after the completion of the reaction was subjected to molecular distillation, followed by water washing and deodorization, and 0.2 mass% of a tocopherol preparation (manufactured by Riken Vitamin Co., Ltd.) was added. Thus, a fat or oil "a" was obtained.
Fat or oil "b": high-oleic safflower oil (manufactured by J-oil Mills, Inc.)
Fat or oil "c": The fat or oil "a" and the fat or oil "b" were mixed at a mass ratio of 1:1 to provide a fat or oil "c".
Fat or oil "d": The fat or oil "a" and the fat or oil "b" were mixed at a mass ratio of 1:4 to provide a fat or oil "d".
The glyceride compositions and fatty acid compositions of the fat or oil "a" to the fat or oil "d" are shown in Table 1.

**Table 1**

| (mass%) | | Fat or oil "a" | Fat or oil "b" | Fat or oil "c" | Fat or oil "d" |
|---|---|---|---|---|---|
| Glyceride composition | TAG | 15.6 | 98.1 | 61.6 | 84.5 |
| | DAG | 83.1 | 1.8 | 37.8 | 15.2 |
| | MAG | 1.1 | 0.1 | 0.4 | 0.2 |
| | Free fatty acids | 0.3 | 0.0 | 0.1 | 0.1 |
| Fatty acid composition of fat or oil | Palmitic acid | 2.5 | 4.8 | 3.7 | 4.4 |
| | Stearic acid | 1.6 | 2.1 | 1.9 | 2.0 |
| | Oleic acid | 23.4 | 78.6 | 52.5 | 68.6 |
| | Linoleic acid | 15.9 | 12.5 | 14.1 | 13.1 |
| | α-Linolenic acid | 55.3 | 0.2 | 26.4 | 10.5 |
| | Others | 1.3 | 1.9 | 1.4 | 1.4 |

| | | | | | |
|---|---|---|---|---|---|
| TAG: Triacylglycerols DAG: Diacylglycerols MAG: Monoacylglycerols | | | | | |

### Vitamin C palmitate (VCP): L-ascorbic acid palmitic acid ester (manufactured by DSM)

Vitamin C stearate (VCS): 6-O-stearoyl-L-ascorbic acid (manufactured by Tokyo Chemical Industry Co., Ltd.)
Sodium citrate: purified sodium citrate (manufactured by Fuso Chemical Co., Ltd., trisodium citrate dihydrate is used as a substance)
Sodium tartrate: sodium L-tartrate (manufactured by Fuso Chemical Co., Ltd.)
Sodium malate: sodium DL-malate (manufactured by Fuso Chemical Co., Ltd.)
Citric acid: purified citric acid (manufactured by Fuso Chemical Co., Ltd.)
Processed starch: Emulster 500A (manufactured by Matsutani Chemical Industry Co., Ltd.)
Casein sodium: casein sodium AR (manufactured by San-Ei Gen F.F.I., Inc.)
Polyglycerin fatty acid ester: Sunsoft Q-17s (manufactured by Taiyo Kagaku Co., Ltd.)
Lysolecithin: SLP-White (manufactured by Tsuji Oil Mills Co., Ltd.) Butter milk powder: Hokkaido butter milk powder (manufactured by Yotsuba Milk Products Co., Ltd.)
Rosemary extract: StabilEnhance OSR D4 (manufactured by Naturex) Hydrophilic antioxidant: XtraBlend RN (manufactured by Naturex) Dextrin: Sandec #100 (manufactured by Sanwa Starch Co., Ltd.)

### Comparative Examples 1 to 6

### [Preparation of Oil-in-water emulsified composition]

A fat or oil and VCP were loaded into a four-necked flask at a ratio shown in Table 2, and the mixture was stirred at 95°C for 30 minutes (300 r/min to 350 r/min) under a nitrogen atmosphere to dissolve the components. Thus, an oil phase was prepared. It was recognized that the oil phase was free from becoming clouded even when left to stand at room temperature for 1 hour after being left to stand to cool.

The components (C) and (D) shown in Table 2 were added to ion-exchanged water, followed by stirring at 10,000 r/min for 2 minutes. Thus, an aqueous phase was prepared. The oil phase was loaded to the aqueous phase, followed by stirring at 10,000 r/min for 8 minutes. Thus, each of oil-in-water emulsified compositions was prepared.

### [Evaluation]

The oil-in-water emulsified compositions prepared above were evaluated by the following three methods.

### (1) Sensory Evaluation

10 g of the oil-in-water emulsified composition was placed into a 20 mL vial bottle with a lid, and was stored at room temperature for 24 hours. After that, three expert panelists performed evaluation in accordance with the following criteria. The score for the "fish odor" and "fishy odor" of Comparative Example 1 was defined as "5" (extremely strongly sensed), and discussion was made by the three panelists to determine the scores for other examples.
5: Fish odor and fishy odor are extremely strongly sensed
4: Fish odor and fishy odor are strongly sensed
3: Fish odor and fishy odor are sensed and regarded as a problem
2: Fish odor and fishy odor are slightly sensed but not regarded as a problem
1: Fish odor and fishy odor are hardly sensed

### (2) Relative Area Ratio of Peak Area of 1-Penten-3-one

The analysis of 1-penten-3-one, which was one of the typical fish odor components, was performed to evaluate suppression effects on fish odor and fishy odor. The analysis of 1-penten-3-one was performed by a headspace solid phase microextraction/gas chromatography/mass spectrometry (HS-SPME/GC/MS) method. 50/30 um, DVB/CAR/PDMS fiber (manufactured by Supelco, Inc.) was used as a SPME fiber.

10 g of the oil-in-water emulsified composition was placed into a 20 mL vial bottle with a lid, and was stored at room temperature for 24 hours. After that, 1 g of a sample was weighed in a vial for a headspace (10 mL), and warmed at 40°C for 20 minutes. After that, a headspace phase was sampled with the SPME fiber for 30 minutes. After that, the analysis was performed under the following conditions.

### <GC/MS Analysis Conditions>

Apparatus: Agilent 7890A/5975 (manufactured by Agilent Technologies)
Column: VF-WAX 60 m × 0.25 mm × 1.0 µm (manufactured by Agilent J&W)
Carrier gas: 1.0 mL He/min
Injector: Splitless, 240°C
Oven temperature: kept at 35°C for 4 min→increased at 3°C/min→185°C→increased at 10°C/min→kept at 240°C for 10 min

A volatile component in a gas phase was analyzed with a HS-GC/MS method using SPME, and the peak area of 1-penten-3-one serving as one of the causal components of fish odor was calculated by integration. Relative ratios of Comparative Examples 2 to 6 when the peak area of Comparative Example 1 was set to 1.00 were determined.

### (3) Oxidative Stability

The evaluation of oxidative stability was performed for compositions having a score of "1" (hardly sensed) or "2" (slightly sensed but not regarded as a problem) in the sensory evaluation. In the evaluation of oxidative stability, 10 g of the oil-in-water emulsified composition was loaded into a 20 mL vial bottle with a lid, and was stored at 60°C for 3 days. After that, an oil of the oil-in-water emulsified composition was extracted and the POV thereof was measured.

The results are shown in Table 2.

**Table 2**

| (mass%) | | | | Comparative Example 1 | Comparative Example 2 | Comparative Example 3 | Comparative Example 4 | Comparative Example 5 | Comparative Example 6 |
|---|---|---|---|---|---|---|---|---|---|
| Composition of oil-in-water emulsified composition | Oil phase | (A) | Fat or oil "a" | 40 | 40 | 40 | 40 | - | - |
| | | | Fat or oil "c" | - | - | - | - | 40 | 40 |
| | | | Ratio of ALA in fatty acid composition of (A) fat or oil | 55.3 | 55.3 | 55.3 | 55.3 | 26.4 | 26.4 |
| | | | DAG in (A) fat or oil | 83.1 | 83.1 | 83.1 | 83.1 | 37.8 | 37.8 |
| | | (B) | VCP* | 0.1 | 0.1 | - | 0.015 | - | 0.1 |
| | Aqueous phase | (C) | Sodium citrate | - | - | - | 5 | - | - |
| | | | Amount obtained by converting fatty acid to free hydroxy acid | - | - | - | 3.27 | - | - |
| | | (D) | Processed starch | 3 | - | 3 | 3 | 3 | 3 |
| | | Water | | Balance | Balance | Balance | Balance | Balance | Balance |
| | ALA in (A) fat or oil/(B) VC fatty acid ester* | | | 553.3 | 553.3 | - | 3,688.9 | - | 263.9 |
| | (C) Amount obtained by converting fatty acid to free hydroxy acid/(B) VC fatty acid ester* | | | - | - | - | 217.8 | - | - |
| | Total | | | 100.0 | 100.0 | 100.0 | 100.0 | 100.0 | 100.0 |
| Odor evaluation | Sensory evaluation | | | 5 | 5 | 1 | 2 | 1 | 3 |
| | Area ratio of 1-penten-3-one peak area (Relative ratio with respect to Comparative Example 1) | | | 1.00 | 0.41 | 0.09 | 0.07 | 0.05 | 0.38 |
| Oxidative stability evaluation | POV after storage at 60°C for 3 days [meq/kg oil] | | | - | - | 17.9 | 17.8 | 19.5 | - |

| | | | | | | | | | |
|---|---|---|---|---|---|---|---|---|---|
| *mass% with respect to fat or oil "a" or fat or oil "c" | | | | | | | | | |

As shown in Table 2, in the compositions of Comparative Examples 1, 2, and 6 each obtained by blending the fat or oil "a" or the fat or oil "c" and 0.1% of VCP had strong fish odor and fishy odor. The compositions of Comparative Example 3 and Comparative Example 5 each obtained by blending no VCP and the composition of Comparative Example 4 obtained by blending 0.015% of VCP had less fish odor and fishy odor, which were each at such a level as to cause no problem, but had low oxidative stability.

### Examples 1 to 23

### [Preparation of Oil-in-water emulsified composition]

A fat or oil and VCP or VCS were loaded into a four-necked flask at a ratio shown in Table 3, and the mixture was stirred at 95°C for 30 minutes (300 r/min to 350 r/min) under a nitrogen atmosphere to dissolve the components. Thus, an oil phase was prepared. It was recognized that the oil was free from becoming clouded even when left to stand at room temperature for 1 hour after being left to stand to cool.

The components (C) and (D) shown in Table 3 were added to ion-exchanged water, followed by stirring at 10,000 r/min for 2 minutes. Thus, an aqueous phase was prepared. The oil phase was loaded to the aqueous phase, followed by stirring at 10,000 r/min for 8 minutes. Thus, each of oil-in-water emulsified compositions was prepared.

### [Evaluation]

The oil-in-water emulsified compositions prepared above were evaluated by the following three methods.

### (1) Sensory Evaluation

The score for the "fish odor" and "fishy odor" of Comparative Example 1 was defined as "5" (extremely strongly sensed), and evaluation was performed in the same manner as in the evaluations of Comparative Examples 2 to 6.

### (2) Relative Area Ratio of Peak Area of 1-Penten-3-one

The peak area of Comparative Example 1 was set to 1.00, and relative area ratios were determined in the same manner as in Comparative Examples 2 to 6.

### (3) Oxidative Stability

The oil-in-water emulsified compositions were stored at 60°C for 3 days in the same manner as in Comparative Example 3 to 5. After that, the POV was measured.

The results are shown in Table 3.

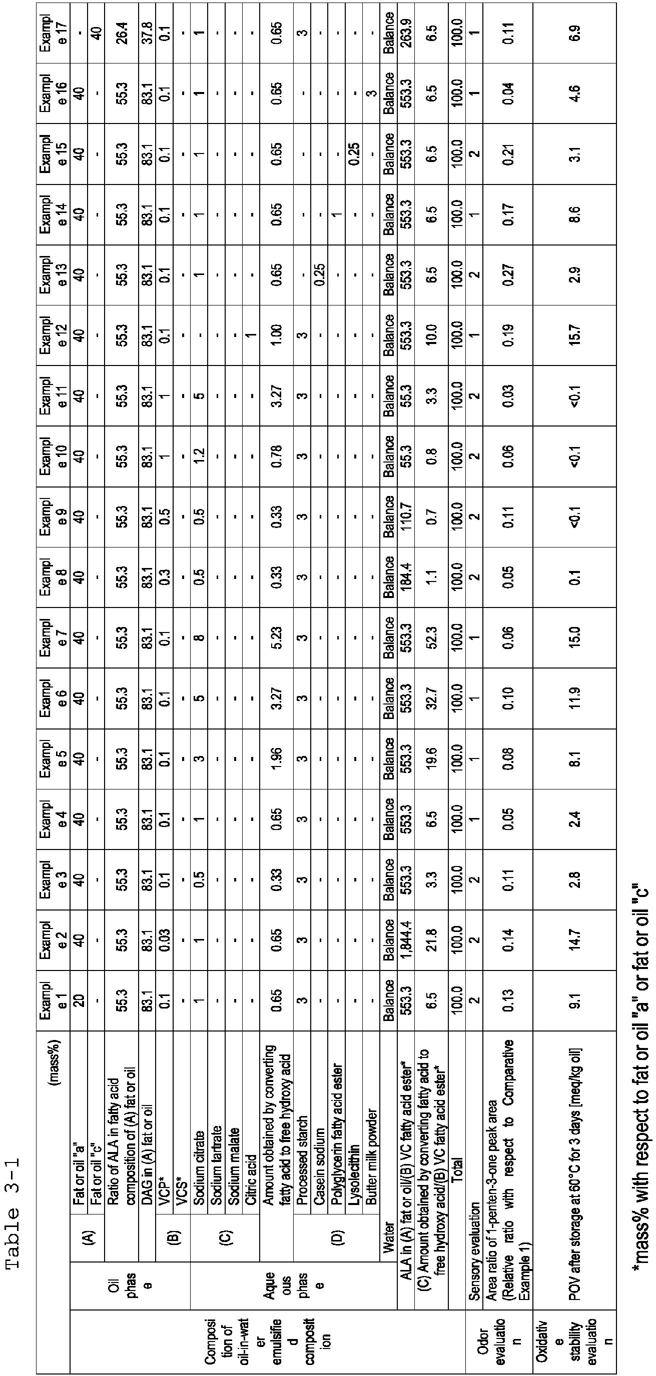

**Table 3-2**

| (mass%) | | | | Example 18 | Example 19 | Example 20 | Example 21 | Example 22 | Example 23 |
|---|---|---|---|---|---|---|---|---|---|
| Composition of oil-in-water emulsified composition | Oil phase | (A) | Fat or oil "a" | 40 | 40 | 40 | 40 | 40 | 40 |
| | | | Fat or oil "c" | - | - | - | - | - | - |
| | | | Ratio of ALA in fatty acid composition of (A) fat or oil | 55.3 | 55.3 | 55.3 | 55.3 | 55.3 | 55.3 |
| | | | DAG in (A) fat or oil | 83.1 | 83.1 | 83.1 | 83.1 | 83.1 | 83.1 |
| | | (B) | VCP* | 0.2 | 0.3 | 0.5 | 0.1 | 0.1 | - |
| | | | VCS* | - | - | - | - | - | 0.1 |
| | Aqueous phase | (C) | Sodium citrate | 1 | 1 | 1 | - | - | 1 |
| | | | Sodium tartrate | - | - | - | 1 | - | - |
| | | | Sodium malate | - | - | - | - | 1 | - |
| | | | Citric acid | - | - | - | - | - | - |
| | | | Amount obtained by converting fatty acid to free hydroxy acid | 0.65 | 0.65 | 0.65 | 0.65 | 0.65 | 0.65 |
| | | (D) | Processed starch | 3 | 3 | 3 | - | - | 3 |
| | | | Casein sodium | - | - | - | - | - | - |
| | | | Polyglycerin fatty acid ester | - | - | - | - | - | - |
| | | | Lysolecithin | - | - | - | - | - | - |
| | | | Butter milk powder | - | - | - | 3 | 3 | - |
| | | Water | | Balance | Balance | Balance | Balance | Balance | Balance |
| | ALA in (A) fat or oil/(B) VC fatty acid ester* | | | 276.7 | 184.4 | 110.7 | 553.3 | 553.3 | 553.3 |
| | (C) Amount obtained by converting fatty acid to free hydroxy acid/(B) VC fatty acid ester* | | | 3.3 | 2.2 | 1.3 | 6.5 | 6.5 | 6.5 |
| | Total | | | 100.0 | 100.0 | 100.0 | 100.0 | 100.0 | 100.0 |
| Odor evaluation | Sensory evaluation | | | 1 | 2 | 2 | 2 | 2 | 1 |
| | Area ratio of 1-penten-3-one peak area (Relative ratio with respect to Comparative Example 1) | | | 0.01 | 0.01 | 0.01 | 0.01 | 0.01 | 0.01 |
| Oxidative stability evaluation | POV after storage at 60°C for 3 days [meq/kg oil] | | | 0.5 | 0.2 | <0.1 | 3.1 | 2.7 | 1.1 |

| | | | | | | | | | |
|---|---|---|---|---|---|---|---|---|---|
| *Mass% with respect to fat or oil "a" or fat or oil "c" | | | | | | | | | |

As shown in Table 3-1 and Table 3-2, the compositions of Examples 1 to 23 each had a score of "1" (hardly sensed) or "2" (slightly sensed but not regarded as a problem) with less fish odor and fishy odor, which were each at such a level as to cause no problem. It found that the relative area ratio of the peak area of 1-penten-3-one with respect to that of Comparative Example 1 was also small. In addition, the oxidative stability was also satisfactory as compared to Comparative Example 3 to 5.

### Examples 24 and 25 and Comparative Example 7

### [Preparation of Oil-in-water emulsified composition]

The fat or oil "a", and VCP and a rosemary extract were heated and mixed with each other at a ratio shown in Table 4 under reduced pressure. Thus, an oil phase was prepared.

The components (C) and (D), a hydrophilic antioxidant, and dextrin shown in Table 4 were added while ion-exchanged water was stirred at 1,400 r/min, followed by stirring at 4,000 r/min for 26 minutes. Thus, an aqueous phase was prepared. The oil phase was loaded to the aqueous phase, and the resultant was stirred at 8,000 r/min for 15 minutes to provide an oil-in-water emulsified composition.

### [Preparation Powdered Fat or oil]

The oil-in-water emulsified composition obtained above was subjected to spray drying with a spray dryer (manufactured by Sakamoto Giken Co., Ltd., TRS-5W2N) under the conditions of a blast temperature of 150°C, a spray flow rate of 90 g/min, a two-fluid nozzle, and a spray air flow rate of from 150 L/min to 160 L/min to provide a powdered fat or oil.

### [Evaluation]

The powdered fats or oils prepared above were evaluated by the following two methods.

### (1) Oxidative Stability

An oil of the powdered fat or oil was extracted, and the POV thereof was measured.

### (2) Sensory Evaluation

Three expert panelists performed evaluation on the taste and flavor of the powdered fat or oil in accordance with the following criteria, and discussion was made by the three panelists to determine the score.
3: Fish odor, fishy odor, and oxidative deterioration odor of oil are strongly sensed
2: Fish odor, fishy odor, and oxidative deterioration odor of oil are sensed and regarded as a problem
1: Fish odor, fishy odor, and oxidative deterioration odor of oil are hardly sensed

The results are shown in Table 4.

**Table 4**

| (mass%) | | | | Example 24 | Example 25 | Comparative Example 7 |
|---|---|---|---|---|---|---|
| Composition of oil-in-water emulsified composition | Oil phase | (A) | Fat or oil "a" | 26 | 26 | 20 |
| | | | Ratio of ALA in fatty acid composition of (A) fat or oil | 55.3 | 55.3 | 55.3 |
| | | | DAG in (A) fat or oil | 83.1 | 83.1 | 83.1 |
| | | (B) | VCP* | 0.025 | 0.1 | - |
| | | Rosemary extract* | | 0.08 | 0.32 | - |
| | Aqueous phase | (C) | Sodium citrate | 0.3 | 0.3 | - |
| | | | Amount obtained by converting fatty acid to free hydroxy acid | 0.20 | 0.20 | - |
| | | (D) | Butter milk powder | 5 | 5 | - |
| | | | Casein sodium | 0.3 | 0.3 | - |
| | | | Processed starch | - | - | 5 |
| | | Hydrophilic antioxidant | | 0.1 | 0.1 | 0.3 |
| | | Dextrin | | 25 | 25 | 15 |
| | | Water | | Balance | Balance | Balance |
| | ALA in (A) fat or oil/(B) VC fatty acid ester* | | | 2,213.3 | 553.3 | - |
| | (C) Amount obtained by converting fatty acid to free hydroxy acid/(B) VC fatty acid ester* | | | 7.84 | 1.96 | - |
| | Total | | | 100.0 | 100.0 | 100.0 |
| Evaluation of powdered fat or oil | POV [meq/kg oil] | | | 0.8 | 0 | 2.3 |
| | Sensory evaluation | | | 1 | 1 | 3 |

| | | | | | | |
|---|---|---|---|---|---|---|
| *mass% with respect to fat or oil "a" | | | | | | |

As shown in Table 4, the oil-in-water emulsified composition of the present invention of each of Examples 24 and 25 had high oxidative stability that enabled the composition to resist heat load at the time of spray drying, and hence the powdered fat or oil obtained by spray drying of the composition was suppressed in fish odor, fishy odor, and oxidative deterioration odor of oil, and hence had satisfactory taste and flavor.

### Reference Examples 1 and 2

### [Preparation of Oil-in-water emulsified composition]

The fat or oil "d" and VCP were loaded into a four-necked flask at a ratio shown in Table 5, and the mixture was stirred at 95°C for 30 minutes (300 r/min to 350 r/min) under a nitrogen atmosphere to dissolve the components. Thus, an oil phase was prepared. It found that the oil was free from becoming clouded even when left to stand at room temperature for 1 hour after being left to stand to cool.

The component (D) shown in Table 5 was added to deionized water, followed by stirring at 10,000 r/min for 2 minutes. Thus, an aqueous phase was prepared. The oil phase was loaded to the aqueous phase, followed by stirring at 10,000 r/min for 8 minutes. Thus, each of oil-in-water emulsified compositions was prepared.

### [Evaluation]

The oil-in-water emulsified compositions prepared above were subjected to sensory evaluation. In the sensory evaluation, the score for the "fish odor" and "fishy odor" of Comparative Example 1 was defined as "5" (extremely strongly sensed), and evaluation was performed in the same manner as in the evaluations of Comparative Examples and Examples described above.

The results are shown in Table 5.

**Table 5**

| (mass%) | | | | Reference Example 1 | Reference Example 2 |
|---|---|---|---|---|---|
| Composition of oil-in-water emulsified composition | Oil phase | (A) | Fat or oil "d" | 40 | 40 |
| | | | Ratio of ALA in fatty acid composition of (A) fat or oil | 10.5 | 10.5 |
| | | | DAG in (A) fat or oil | 15.2 | 15.2 |
| | | (B) | VCP* | - | 0.1 |
| | Aqueous phase | (C) | Sodium citrate | - | - |
| | | | Amount obtained by converting fatty acid to free hydroxy acid | - | - |
| | | (D) | Processed starch | 3 | 3 |
| | | Water | | Balance | Balance |
| | ALA in (A) fat or oil/(B) VC fatty acid ester* | | | - | 105.48 |
| | (C) Amount obtained by converting fatty acid to free hydroxy acid/(B) VC fatty acid ester* | | | - | - |
| | Total | | | 100.0 | 100.0 |
| Odor evaluation | Sensory evaluation | | | 1 | 1 |

| | | | | | |
|---|---|---|---|---|---|
| *mass% with respect to fat or oil "d" | | | | | |

As shown in Table 5, in the fat or oil "d" having an ALA concentration of 10.5%, even when VCP was added, the oil-in-water emulsified composition had sufficiently low fish odor and fishy odor which was acceptable, and hence no problem occurred.

## Claims

1. An oil-in-water emulsified composition, comprising the following components (A), (B), and (C) :
(A) a fat or oil having a content of diacylglycerols of 15 mass% or more and a content of α-linolenic acid in constituent fatty acids of the fat or oil of 15 mass% or more;
(B) 0.020 mass% to 1.0 mass% of a vitamin C fatty acid ester with respect to the component (A); and
(C) 0.15 mass% to 6.0 mass% of a hydroxy acid or a salt thereof.

2. The oil-in-water emulsified composition according to claim 1, wherein (C) the hydroxy acid or the salt thereof is one or more selected from the group consisting of citric acid, tartaric acid, malic acid, and salts thereof.

3. The oil-in-water emulsified composition according to claim 1 or 2, further comprising (D) an emulsifying agent.

4. The oil-in-water emulsified composition according to claim 3, wherein (D) the emulsifying agent is one or more selected from the group consisting of processed starch, a polyglycerin fatty acid ester, lecithin or an enzymatic degradation product thereof, and milk proteins.

5. The oil-in-water emulsified composition according to any one of claims 1 to 4, wherein the oil-in-water emulsified composition has a mass ratio of an oil phase to an aqueous phase, oil phase/aqueous phase, of from 5/95 to 80/20.

6. A powdered fat or oil, which is obtained by drying the oil-in-water emulsified composition of any one of claims 1 to 5.
